(19) Europäisches Patentamt
European Patent Office
Office européen des brevets

(11) Numéro de publication : **0 445 021 A2**

# (12) DEMANDE DE BREVET EUROPEEN

(21) Numéro de dépôt : **91400517.8**

(22) Date de dépôt : **26.02.91**

(51) Int. Cl.⁵ : **C07H 17/04**, C07D 493/04, C07H 13/04, C07H 13/10

(30) Priorité : **27.02.90 FR 9002408**

(43) Date de publication de la demande : **04.09.91 Bulletin 91/36**

(84) Etats contractants désignés : **AT BE CH DE DK ES FR GB GR IT LI LU NL SE**

(71) Demandeur : **PIERRE FABRE MEDICAMENT**
**45, Place Abel Gance**
**F-92100 Boulogne (FR)**

(72) Inventeur : **Robin, Jean-Pierre**
**28 rue Sainte-Croix**
**F-72000 Le Mans (FR)**
Inventeur : **Lenain, Valéry**
**28 rue Sainte-Croix**
**F-72000 Le Mans (FR)**

(74) Mandataire : **Warcoin, Jacques et al**
**Cabinet Régimbeau 26, avenue Kléber**
**F-75116 Paris (FR)**

(54) **Tris acétyl-2",3",4' éthylidène-4",6"beta-D-glucopyranosides, leur préparation et leur utilisation pour la préparation d'éthylidène beta-D-glucopyranoside de déméthyl-4' épipodophyllotoxine.**

(57) L'invention concerne des Tris acétyl-2",3",4' éthylidène-4",6" β-D-glucopyranosides, leur préparation et leur utilisation pour la préparation d'éthylidène β-D-glucopyranoside de déméthyl-4' épipodophyllotoxine.

Les composés de l'invention répondent à la formule 1

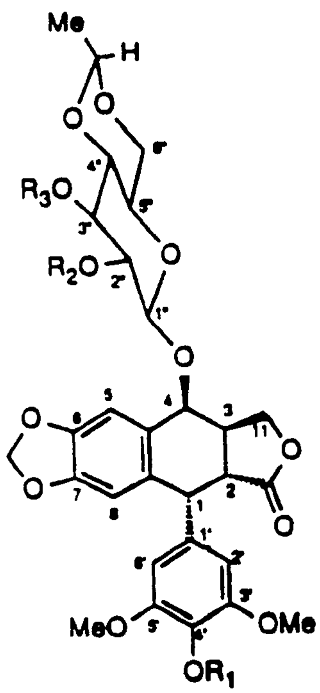

1

Leur procédé de préparation comporte les étapes suivantes :
— acylation d'un dérivé glycosylé
— acylation d'une aglycone
— couplage du dérivé glycosylé acylé et de l'aglycone acylée, en présence d'un acide de Lewis, l'acylation étant effectuée à l'aide d'un chlorure d'acide, éventuellement identique pour les deux étapes d'acylation, dans lequel le carbone en α de la fonction carbonyle porte au moins un hétéroatome choisi parmi O et S.

EP 0 445 021 A2

# TRIS ACETYL-2",3",4' ETHYLIDENE-4",6" β-D-GLUCOPYRANOSIDES, LEUR PREPARATION ET LEUR UTILISATION POUR LA PREPARATION D'ETHYLIDENE β-D-GLUCOPYRANOSIDE DE DEMETHYL-4' EPIPODOPHYLLOTOXINE

La présente invention a pour objet des tris acétyl-2",3",4' éthylidène-4",6" β-D-glucopyranosides de déméthyl-4' épipodophyllotoxine utiles pour la préparation d'éthylidène β-D-glucopyranoside de déméthyl-4' épipodophyllotoxine connu comme médicament, un procédé pour leur préparation à partir d'esters de dérivés glycosylés et d'aglycone, ainsi que les esters de dérivés glycosylés et d'aglycone utilisés.

L'éthylidène glucoside de déméthyl-4' épipodophyllotoxine est connu comme étant un principe actif d'intérêt majeur en thérapeutique anticancéreuse humaine. Ce composé répond à la formule 1'

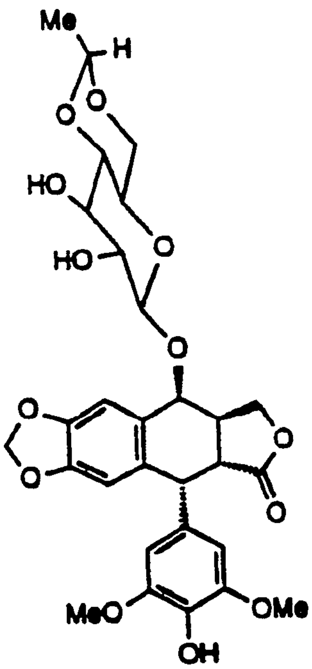

1'

Différents procédés de préparation de ce composé sont connus. Ils consistent en général à coupler un intermédiaire glycosylé dont les hydroxyles en position 4 et 6 sont bloqués sous forme d'un acétal cyclique et les hydroxyles en position 2 et 3 sont protégés, avec un aglycone dont l'hydroxyle en 4' est également protégé, puis à effectuer la déprotection du composé obtenu par la réaction de couplage.
L'intermédiaire glycosylé est représenté par la formule 2

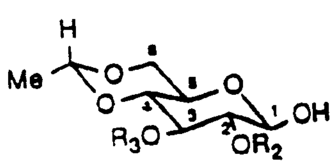

2

dans laquelle $R_2$ et $R_3$ représentent les groupements protecteurs des hydroxyles en position 2 et 3.
L'aglycone est représentée par la formule 3

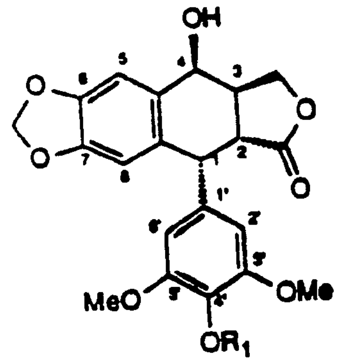

3

dans laquelle $R_1$ représente le groupement protecteur de l'hydroxyle en position 4'.
Le composé issu du couplage d'un composé 2 avec un composé 3 est représenté par la formule 1

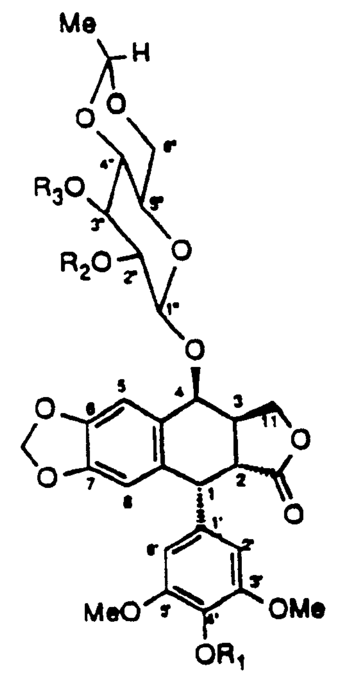

1

Une première voie de synthèse, décrite dans la littérature, utilise des acétates pour protéger l'intermédiaire glycosylé et un benzyloxycarbonyle pour l'aglycone. Dans le composé 1 obtenu, $R_2 = R_3 = Ac$, $R_1 = CO_2Bn$ [M. Kuhn, and A. Von Wartburg, Helv. Chim. Acta, 52, 948-955 (1969)]. Elle présente deux inconvénients : a) nécessité de deux étapes de déblocage successives pour chaque type de groupement protecteur, b) trop grande stabilité des esters acétiques nécessitant des temps de réaction prolongés, conduisant à une proportion de produits secondaires intolérable à l'étape de déblocage ( anomérisation et ouverture du cycle lactonique par transestérification).

Une deuxième voie décrite utilise le même intermédiaire glycosylé, mais avec des formiates en lieu et place des acétates. Dans le composé 1 obtenu $R_2 = R_3 = CHO$, $R_1 = CO_2Bn$ [M. Kuhn, C. Keller- Juslen, J. Renz et A. Von Wartburg, ( Sandoz Ltd) CH-518927 ( Cl. CO7d), 15 dec 1971, 2845/68, 27 Fev 1968]. Cette méthode présente également l'inconvénient de nécessiter un déblocage spécial du carbonate situé en 4'. D'autre part, contrairement aux esters acétiques de la premiere voie, ces esters formiques sont si labiles qu' ils ne permettent pas une conservation des intermédiaires glycosylés, ce qui est un inconvénient lors de la mise en oeuvre du procédé à l'échelle industrielle.

Une troisème voie, décrite plus récemment [ JP 58,219,196 (83,219,196; JP 58.225.096 (83,225,096); EP

111,058; JP 101,766; EP 196,618; JP 61,122,294 (86,122,294); JP 61,103,883 (86,103,883)] utilise des composés du type de 1, mais comportant des α-haloacétates pour protéger les hydroxyles en 2 et 3 de la partie glycosylée et des β-haloalcoxycarbonates pour protéger l'hydroxyle en 4' de l'aglycone, soit : $R_1$ = $CX_mH_nCH_2OCO$-, $R_2$ = $R_3$ = $CX_mH_nCO$-, avec m + n = 3 et X = F, Cl, Br, I) ou bien encore des α-haloacétates pour protéger les trois sites, soit : $R_1$ = $R_2$ = $R_3$ = $CX_mH_nCO$-, avec m + n = 3 et X = F, Cl, Br, I. Cette méthode présente l'inconvénient lors de la déprotection par hydrogénolyse, d'utiliser des intermédiaires glycosylés comportant une différence de réactivité trop faible en 2 et 3 par rapport au groupement protecteur benzyloxycarbonyle de l'hydroxyle anomérique, en position 1. D'autre part, la présence d'esters d'acides organiques forts protégeant l'hydroxyle adjaçant à l'hydroxyle hémiacétalique, conduit, en raison de l'effet anomérique, à une isomérisation plus difficile à contrôler. La mauvaise stabilité en résultant ne permet pas, en outre, un stockage prolongé des intermédiaires glycosylés sous la forme anomérique souhaitée (béta), ce qui est un inconvénient majeur en vue de l'utilisation thérapeutique du produit final. De plus, le contrôle analytique de la pureté anomérique des intermédiaires glycosylés 2, dans lesquels $R_2$ = $R_3$ = $CX_mH_nCO$-, $CX_mH_n$ $CH_2OCO$- avec m + n = 3, X = F, Cl, Br, I, est plus difficile à mettre en oeuvre, car ces intermédiaires ne comportent pas de chromophore absorbant en lumière ultraviolette. Par ailleurs, ce procédé présente l'inconvénient de nécessiter des conditions sophistiquées pour le blocage sélectif de l'hydroxyle phénolique de l'aglycone ( basse température), en raison de la forte réactivité des réactants acylants utilisés vis à vis des deux types de sites. Enfin, la plupart des groupements décrits sont très instables (iodoacétate, bromoacétate, trifluoroacétate) et ne permettent pas d'isoler les intermédiaires.

Le but de la présente invention est de supprimer ces inconvénients, c' est-à-dire de minimiser la formation de produits indésirables lors de la déprotection du composé 1 pour obtenir le composé 1', d'accroître la stabilité et la pureté des intermédiaires glycosylés 2, de faciliter le contrôle analytique de la pureté des intermédiaires, et de permettre la déprotection du composé 1 en une seule étape.

Ce but est atteint par un procédé de préparation des composés 1 mettant en oeuvre des réactifs particuliers pour protéger les hydroxyles en position 2 et 3 des intermédiaires glycosylés 2 d'une part, l'hydroxyle en position 4' de l'aglycone 3 d'autre part.

La présente invention a pour objet un procédé de préparation de tris acétyl-2", 3", 4' éthylidène-4", 6" β-D-glucopyranosides de déméthyl-4' épipodophyllotoxine 1, et les composés 1 obtenus.

L'invention a également pour objet les intermédiaires glycosylés 2 et les aglycones 3 obtenus comme intermédiaires de synthèse lors de la mise en oeuvre du procédé de l'invention.

Enfin, l'invention a pour objet l'application des composés 1 à la préparation d'éthylidène glucoside de déméthyl-4' épipodophyllotoxine.

Selon l'invention, le procédé de préparation de composés répondant à la formule 1

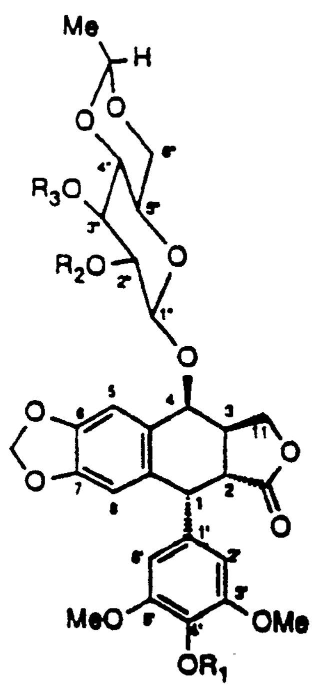

1

dans laquelle $R_1$, $R_2$ et $R_3$, identiques ou différents, représentent des radicaux acyles dans lesquels le carbone en α de la fonction carbonyle porte au moins un hétéroatome choisi parmi O et S, est caractérisé en ce qu' il comporte les étapes suivantes :

1) préparation d'un dérivé glycosylé répondant à la formule 2,

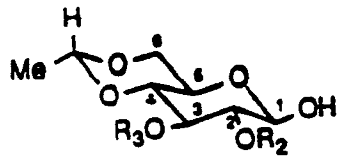

2

dans laquelle $R_2$ et $R_3$ ont la signification donnée ci-dessus, par réaction d'au moins un chlorure d'acide $R_2Cl$ et/ou $R_3Cl$ dans lesquels le carbone en α de la fonction carbonyle porte au moins un hétéroatome choisi parmi O et S avec un dérivé glycosylé répondant à la formule 2',

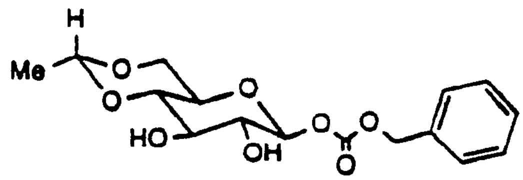

2'

suivie d'une hydrogénolyse catalysée par du charbon palladié.

2) préparation d'une aglycone répondant à la formule 3,

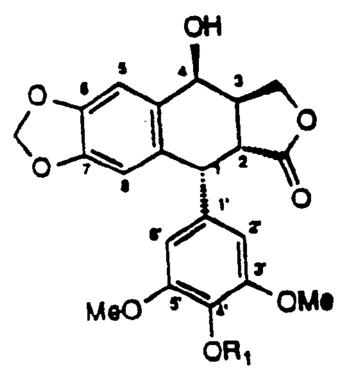

3

dans laquelle $R_1$ a la signification donnée ci-dessus, par réaction de déméthyl-4' épipodophyllotoxine 3'

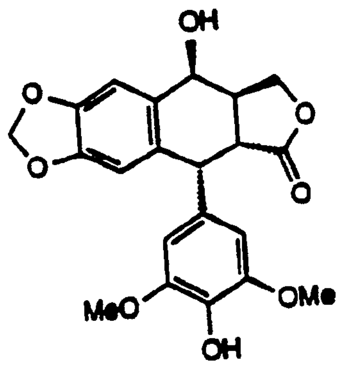

3'

avec un chlorure d'acide $R_1Cl$ dans lequel le carbone en α de la fonction carbonyle porte au moins un hétéroatome choisi parmi O et S, la réaction étant effectuée en milieu neutre; l'ordre dans lequel les étapes 1) et 2) sont effectuées étant indifférent.

3) couplage d'un dérivé glycosylé 2 avec une aglycone 3, en présence d'un acide de Lewis en solution dans un hydrocarbure chloré.

Les chlorures d'acide $R_1Cl$, $R_2Cl$, $R_3Cl$ utilisés dans les étapes 1 et 2 peuvent être identiques ou différents. De préférence, l'acylation du composé 2' est effectuée par un chlorure d'acide unique, c' est-à-dire $R_2 = R_3$.

Parmi les radicaux acyles $R_1$, $R_2$ ou $R_3$ appropriés, on peut citer ceux qui répondent aux formules **4a à 4h** suivantes

(4a)

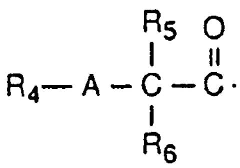

(4b)

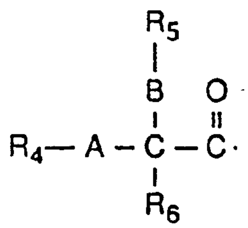

(4c)

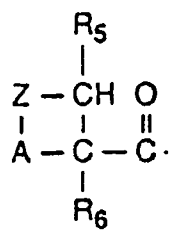

(4d)

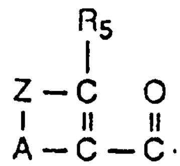

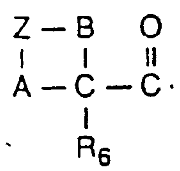

(4e)

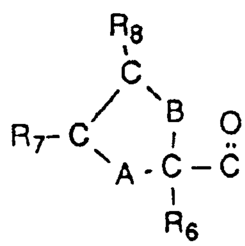

(4f)

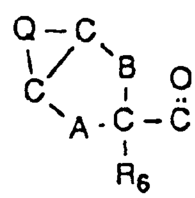

(4g)

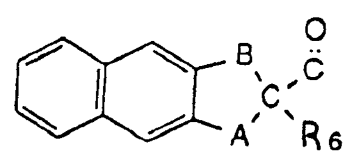

(4h)

dans lesquelles

– A et B, identiques ou différents, représentent chacun un hétéroatome divalent d'oxygène et/ ou de soufre,

– $R_4$, $R_5$, $R_6$, $R_7$, $R_8$, identiques ou différents, représentent chacun, soit un atome d'hydrogène, soit un radical alkyle ayant de 1 à 8 atomes de carbone, saturé, monoinsaturé ou polyinsaturé, linéaire ou ramifié, soit un radical alkyle choisi parmi les motifs suivants : Ar-$(CH_2)_n$- (où n est un nombre entier égal à 1 ou 2, et Ar est un benzène, un naphtalène ou un anthracène), $Ar_2CH$- ( où Ar est un benzène ou un naphtalène), soit un noyau aromatique choisi parmi les motifs répondant aux formules 5a à 5c suivantes

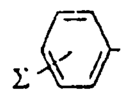 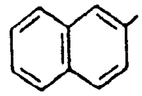 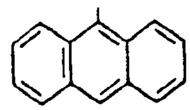

(5a) (5b) (5c)

où Σ représente un ensemble de 1 à 4 substituants identiques ou différents choisis parmi les motifs ou atomes suivants : OMe, OEt, F, Cl, Br, I, $NO_2$.

– Z et Q identiques ou différents, représentent chacun un motif divalent choisi parmi $-(CH_2)_n-$, $-CH(CH_3)-CH_2-$, $-CH_2-CH(CH_3)-$, $-CH(CH_3)-CH(CH_3)-$, $-CH(C_2H_5)-CH_2-$, $-CH_2-CH(C_2H_5)-$, $-C(CH_3)=CH-$, $-CH=CH-(CH_2)_m$, $-CH=CH-CH=CH-$, n étant un nombre entier allant de 1 à 4 inclus et m un nombre entier égal à 1 ou 2, un radical 1,2-phénylène, 2,3-naphtylène, 2,3-anthrylène, un motif tétravalent répondant à la formule suivante:

$=C(R_9)-CR_{10}=CR_{11}-CR_{12}=$, où $R_9$, $R_{10}$, $R_{11}$ et $R_{12}$ sont des radicaux alkyles ayant de 1 à 6 atomes de carbone ou des hétéroatomes d'oxygène, de soufre, d'halogènes (F, Cl, Br, I), ou d'azote.

Ces radicaux acyles comprennent notamment les alcoxyacétyles $R_4-OCH_2-C=0$, par exemple ceux pour lesquels $R_4$ est un méthyle, un éthyle, un benzyle ou un menthyle.

Les radicaux acyles comprennent également les aryloxyacétyles $R_4-OCH_2-C=0$, par exemple ceux pour lesquels $R_4$ est un noyau phényle, naphtyle, anthryle ou phénanthryle, ces noyaux portant éventuellement de 1 à 4 substituants choisis parmi OMe, OEt, F, Cl, Br, I, $NO_2$.

Parmi les radicaux acyles sont également inclus les thioalcoylacétyles $R_4-SCH_2-C=0$, pour lesquels $R_4$ peut être notamment un méthyle ou un benzyle.

D'autres radicaux acyles sont les thiorarylacétyles $R_4-S-CH_2-C=0$, dans lesquels $R_4$ peut être notamment un phényle, un naphtyle, un anthryle ou un phénanthryle.

Un autre groupe de radicaux acyles appropriés comprend les arylènes dioxy-2,2 acétyles Z 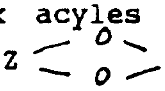 CH-C=0, dans lesquels Z représente par exemple un radical 1,2-phénylène, un radical 2,3-naphtylène, un radical 2,3-anthrylène.

Un autre groupe de radicaux acyles appropriés comprend les radicaux 2-furoyle ou 2-thénoyle.

Pour la première étape du procédé selon l'invention, on fait réagir le composé 2' avec le chlorure d'acide approprié dans un solvant haloalkylé et l'on obtient un composé intermédiaire répondant à la formule 6,

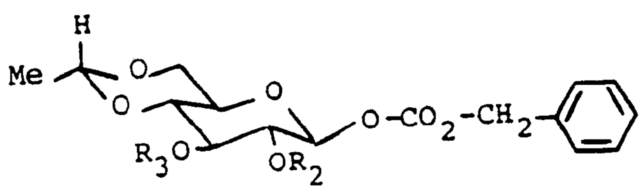

6

dans laquelle $R_2$ et $R_3$ ont la signification donnée précédemment.

puis on effectue l'hydrolyse du groupement benzyloxycarbonyl-1 du composé 6 en présence d'hydrogène moléculaire, en utilisant du charbon palladié comme catalyseur. Cette hydrogénolyse est effectuée sous la pression atmosphérique, à une température comprise entre -10°C et 0°C, dans un solvant cétonique.

Le solvant haloalkylé peut être choisi parmi le dichlorométhane, le dichlorométhane et le chloroforme.

Le solvant cétonique peut être choisi parmi l'acétone, la méthyl-éthylcétone ou la méthyl-iso-butyl cétone.

Le composé intermédiaire obtenu peut être isolé et purifié avant hydrogénolyse. Il peut également être utilisé à l'état brut pour l'hydrogénolyse.

Le produit obtenu après hydrogénolyse est un dérivé glycosylé répondant à la formule 2 dans laquelle les radicaux $R_2$ et $R_3$ ont la signification donnée précédemment.

Pour la deuxième étape du procédé selon l'invention, on fait réagir la déméthyl-4' épipodophyllotoxine 3' avec un chlorure d'acide tel que défini précédemment. La réaction est effectuée en milieu neutre. Le chlorure d'acide est ajouté suffisamment lentement pour être constamment en défaut de concentration par rapport au

composé 3', de manière à limiter la réaction du chlorure d'acide avec l'hydroxyle en position 4 sur le composés 3'. Le déroulement de la réaction est suivi par contrôle de la température du milieu réactionnel et par chromatographie sur couches minces.
Les aglycones 3 obtenues présentent un excellent pouvoir cristallogène, ce qui rend leur purification aisée.
Ils répondent à la formule 3 dans laquelle $R_1$ a la signification donnée précédemment.

Pour la troisième étape du procédé selon l'invention, la température de réaction est maintenue entre -25°C et -20°C, pour limiter la formation de l'anomère α indésirable.
L'hydrocarbure chloré est avantageusement choisi parmi le dichlorométhane ou le dichloroéthane.

Les composés 1 ainsi obtenus sont utiles pour la préparation de l'éthylidène-4",6" β-D-glucopyranoside de déméthyl-4' épipodophyllotoxine 1'.
Le procédé de préparation de 1' consiste à effectuer une transestérification du composé 1 par un alcool à bas point d'ébullition, en présence d'un catalyseur de transestérification. La réaction est effectuée dans un co-solvant -du composé 1 et dudit alcool-, par exemple le tétrahydrofuranne. Un alcool approprié est le méthanol.
Le mélange réactionnel est porté au reflux du solvant pendant une durée comprise entre 1 et 2 heures.
Les groupements retirés du composé 1 forment, avec l'alcool utilisé, un ester qui est séparé avec les autres impuretés mineures, au cours de l'opération finale de purification par chromatographie.

La présente invention est expliquée plus en détails par les exemples ci-après, donnés à titre illustratif, mais non limitatif.

Les exemples 1 à 20 illustrent la préparation de divers dérivés glycosylés répondant à la formule 2.

## EXEMPLE 1

**Bis(phénoxy-2 acétyl)-2,3 éthylidène-4,6 β-D-glucose 2a.**

Dans un mélange de dichlorométhane sec (25 l) et de pyridine (1,41 l), on a introduit sous agitation du benzyloxycarbonyl-1 éthylidène-4,6 β-D-glucose (2,5 kg, soit 7,35 moles) et le milieu réactionnel a été refroidi à 0°C. Du chlorure de phénoxy-2 acétyle (2,45 l, soit 17,64 moles) a été additionné et la réaction s'est poursuivie pendant 1h30 à 20°C. Après contrôle par chromatographie en couche mince, de l'eau a été ajoutée, et la phase organique a été lavée avec une solution de bicarbonate de sodium, séchée sur $MgSO_4$, puis évaporée sous pression réduite. On a incorporé l'huile obtenue à du méthanol chaud (7 l) et on a obtenu 2,7 kg de l'intermédiaire benzyloxycarbonyl-1 (phénoxy-2 acétyl)-2,3 éthylidène-4,6 β-D-glucose 6a (Rdt=60%). Un échantillon a été recristallisé. Son analyse structurale a donné les résultats suivants : PF=128-130°C; $[\alpha]_D^{22}$=-18° (c=1; $CHCl_3$); IR (Nujol)( $\nu$ max); 1769 (C=O) 1600, 1377 et 1198; RMN$^1$H 400 MHz ( CDCl3)(δ ppm): 7,34 (5H, m, H arom.); 7,21 (5H, m, H arom.); 6,92 (2H, m, H arom.); 6,84 et 6,80 (4H, 2d, J=7,9 Hz, H arom.); 5,63 ( 1H, d, $J_{1''-2''}$=8,1 Hz, H-1"$_{(ax)}$; 5,35 ( 1H, t, $J_{3''-2'', 4''}$=9 Hz, H-3"); 5,18 ( 1H, H-3"; 5,17 et 5,14 (2H, 2d, $J_{AB}$=12,0 Hz, $CH_2$-OCO); 4,64 ( 1H, q, $J_{7''-8''}$=5,0 Hz, H-7"); 4,59 et 4,54 (2H, 2d, $J_{AB}$=16,5 Hz, $CH_2$-O); 4,52 et 4,46 (2H, 2d, $J_{AB}$=16,5 Hz, $CH_2$-O); 4,22 ( 1H, m, H-6"$_{(A)}$); 3,52 (3H, m, H-6"$_{(B)}$, H-4" et H-5") et 1,30 (3H, d, $J_{8''-7''}$=5 Hz, H-8").
Dans de l'acétone sèche (9,5 l), on a introduit du charbon palladié à 10% (125 g) et le benzyloxycarbonyl-1 (phénoxy-2 acétyl)-2,3 éthylidène-4,6 β-D-glucose brut (1,73 kg, soit 2,85 moles), ci-dessus. Le mélange a été violemment agité et refroidi à -5°C. Après dissolution, de l'hydrogène a été absorbé à pression atmosphérique, pendant 1h. Le milieu réactionnel a ensuite été filtré, puis évaporé sous pression réduite à 30-35°C. Le bis( phénoxy-2 acétyl)-2,3 éthylidène-4,6 β-D-glucose 2a a été obtenu sous forme d'une huile incolore, conservée à -15°C. Le rendement est de 48%.
Un échantillon a été recristallisé, son étude analytique a donné les résultats suivants: PF = 66-70°C; $[\alpha]_D^{22}$= -1° (c = 1, $CHCl_3$); IR ($CHCl_3$)( $\nu$ max): 1776 (C=O), 1609 et 1497 $cm^{-1}$.

## EXEMPLE 2

**Bis(naphtoxy-2 acétyl)-2,3 éthylidène-4,6 β-D-glucose 2b.**

On a repris le mode opératoire de l'exemple 1, mais en utilisant du chlorure de naphtoxy-2 acétyle, en lieu et place du chlorure de phénoxy-2 acétyle, et pendant un temps de réaction de 8 h, pour obtenir l'intermédiaire 6b. Cet intermédiaire 6b a été directement hydrogénolysé à l'état brut pour donner le produit 2b sous forme d'une huile incolore, avec un rendement global de 29%. Le produit 2b a été conservé tel quel à -15°C.

## EXEMPLE 3

**Bis [(méthoxy-2 phénoxy)-2 acétyl]-2,3 éthylidène-4,6 β-D-glucose 2c.**

On reprend le mode opératoire de l'exemple 1, mais en utilisant du chlorure de ( méthoxy-2 phénoxy)-2 acétyle, en lieu et place du chlorure de phénoxy-2 acétyle, et pendant un temps de réaction de 3 h, pour obtenir l'intermédiaire **6c** . Cet intermédiaire **6c** a été directement hydrogénolysé à l'état brut pour donner le produit **2c** sous forme d'une huile incolore, avec un rendement global de 49%. Le produit **2c** a été conservé tel quel à -15°C.

EXEMPLE 4

**Bis [(méthoxy-4 phénoxy)-2 acétyl]-2,3 éthylidène-4,6 β-D-glucose 2d.**
On reprend le mode opératoire de l'exemple 1, mais en utilisant du chlorure de (méthoxy-4 phénoxy)-2 acétyle, en lieu et place du chlorure de phénoxy-2 acétyle, et pendant un temps de réaction de 4 h, pour obtenir l'intermédiaire **6d**. Cet intermédiaire **6d** a été directement hydrogénolysé a l'état brut pour donner le produit **2d** sous forme d'une huile incolore, avec un rendement global de 37%. Le produit **2d** a été conservé tel quel à -15°C.

EXEMPLE 5

**Bis[(nitro-2 phénoxy)-2 acétyl]-2,3 éthylidène-4,6 β-D-glucose 2e.**
On reprend le mode opératoire de l'exemple 1, mais en utilisant du chlorure de (nitro-2 phénoxy)-2 acétyle, en lieu et place du chlorure de phénoxy-2 acétyle, et pendant un temps de réaction de 8 h, pour obtenir l'intermédiaire **6e**. Cet intermédiaire **6e** a été directement hydrogénolysé à l'état brut pour donner le produit **2e** sous forme d'une huile incolore, avec un rendement global de 38%. Le produit **2e** a été conservé tel quel à -15°C.

EXEMPLE 6

**Bis [(nitro-4 phénoxy)-2 acétyl]-2,3 éthylidène-4,6 β-D-glucose 2f.**
On reprend le mode opératoire de l'exemple 1, mais en utilisant du chlorure de (nitro-4 phénoxy)-2 acétyle, en lieu et place du chlorure de phénoxy-2 acétyle, et pendant un temps de réaction de 8 h, pour obtenir l'intermédiaire **6f**. Cet intermédiaire **6f** a été directement hydrogénolysé à l'état brut pour donner le produit **2f** sous forme d'une huile incolore, avec un rendement global de 40%. Le produit **2f** a été conservé tel quel à -15°C.

EXEMPLE 7

**Bis [(chloro-4 phénoxy)-2 acétyl]-2,3 éthylidène-4,6 β-D-glucose 2q.**
On reprend le mode opératoire de l'exemple 1, mais en utilisant du chlorure de (chloro-4 phénoxy)-2 acétyle, en lieu et place du chlorure de phénoxy-2 acétyle, et pendant un temps de réaction de 8 h, pour obtenir l'intermédiaire **6q**. Cet intermédiaire **6q** a été directement hydrogénolysé à l'état brut pour donner le produit **2q** sous forme d'une huile incolore, avec un rendement global de 31%. Le produit **2q** a été conservé tel quel à -15°C.

EXEMPLE 8

**Bis [(dichloro-2,4 phénoxy)-2 acétyl]-2,3 éthylidène-4,6 β-D-glucose 2h.**
On reprend le mode opératoire de l'exemple 1, mais en utilisant du chlorure de (dichloro-2,4 phénoxy)-2 acétyle, en lieu et place du chlorure de phénoxy-2 acétyle, et pendant un temps de réaction de 8 h, pour obtenir l'intermédiaire **6h**. Cet intermédiaire **6h** a été directement hydrogénolysé à l'état brut pour donner le produit **2h** sous forme d'une huile incolore, avec un rendement global de 46%. Le produit **2h** a été conservé tel quel à -15°C.

EXEMPLE 9

**Bis [(trichloro-2,4,5 phénoxy)-2 acétyl]-2,3 éthylidène-4,6 β-D-glucose 2i.**
On reprend le mode opératoire de l'exemple 1, mais en utilisant du chlorure de (trichloro-2,4,5 phénoxy)-2 acétyle, en lieu et place du chlorure de phénoxy-2 acétyle, et pendant un temps de réaction de 10 h, pour obtenir l'intermédiaire **6i**. Cet intermédiaire **6i** a été directement hydrogénolysé à l'état brut pour donner le produit **2i** sous forme d'une huile incolore, avec un rendement global de 18%. Le produit **2i** a été conservé tel quel à -15°C.

EXEMPLE 10

**Bis [(trichloro-2,4,6 phénoxy)-2 acétyl]-2,3 éthylidène-4,6 β-D-glucose 2j.**
On reprend le mode opératoire de l'exemple 1, mais en utilisant du chlorure de (trichloro-2,4,6 phénoxy)-2 acétyle, en lieu et place du chlorure de phénoxy-2 acétyle, et pendant un temps de réaction de 11 h, pour obtenir

l'intermédiaire 6j. Cet intermédiaire 6j a été directement hydrogénolysé à l'état brut pour donner le produit 2j sous forme d'une huile incolore, avec un rendement global de 26%. Le produit 2j a été conservé tel quel à -15°C.

EXEMPLE 11

**Bis [(fluoro-2 phénoxy)-2 acétyl]-2,3 éthylidène-4,6 β-D-glucose 2k.**
On reprend le mode opératoire de l'exemple 1, mais en utilisant du chlorure de (fluoro-2 phénoxy)-2 acétyle, en lieu et place du chlorure de phénoxy-2 acétyle, et pendant un temps de réaction de 5 h, pour obtenir l'intermédiaire 6k. Cet intermédiaire 6k a été directement hydrogénolysé à l'état brut pour donner le produit 2k sous forme d'une huile incolore, avec un rendement global de 32%. Le produit 2k a été conservé tel quel à -15°C.

EXEMPLE 12

**Bis (méthoxy-2 acétyl)-2,3 éthylidène-4,6 β-D-glucose 2l.**
On reprend le mode opératoire de l'exemple 1, mais en utilisant du chlorure de méthoxy-2 acétyle, en lieu et place du chlorure de phénoxy-2 acétyle, et pendant un temps de réaction de 5 h, pour obtenir l'intermédiaire 6l. Cet intermédiaire 6l a été directement hydrogénolysé à l'état brut pour donner le produit 2l sous forme d'une huile incolore, avec un rendement global de 41%. Le produit 2l a été conservé tel quel à -15°C.

EXEMPLE 13

**Bis (éthoxy-2 acétyl)-2,3 éthylidène-4,6 β-D-glucose 2m.**
On reprend le mode opératoire de l'exemple 1, mais en utilisant du chlorure d'éthoxy-2 acétyle, en lieu et place du chlorure de phénoxy-2 acétyle, et pendant un temps de réaction de 7 h, pour obtenir l'intermédiaire 6m. Cet intermédiaire 6m a été directement hydrogénolysé à l'état brut pour donner le produit 2m sous forme d'une huile incolore, avec un rendement global de 35%. Le produit 2m a été conservé tel quel à -15°C.

EXEMPLE 14

**Bis(benzoxy-2 acétyl)-2,3 éthylidène-4,6 β-D-glucose 2n.**
On reprend le mode opératoire de l'exemple 1, mais en utilisant du chlorure de benzoxy-2 acétyle, en lieu et place du chlorure de phénoxy-2 acétyle, et pendant un temps de réaction de 8h, pour obtenir l'intermédiaire 6n. Cet intermédiaire 6n a été directement hydrogénolysé à l'état brut pour donner le produit 2n sous forme d'une huile incolore, avec un rendement global de 37%. Le produit 2n a été conservé tel quel à -15°C.

EXEMPLE 15

**Bis (menthoxy-2 acétyl)-2,3 éthylidène-4,6 β-D-glucose 2o.**
On reprend le mode opératoire de l'exemple 1, mais en utilisant du chlorure de menthoxy-2 acétyle, en lieu et place du chlorure de phénoxy-2 acétyle, et pendant un temps de réaction de 16h, pour obtenir l'intermédiaire 6o. Cet intermédiaire 6o a été directement hydrogénolysé à l'état brut pour donner le produit 2o sous forme d'une huile incolore, avec un rendement global de 24%. Le produit 2o a été conservé tel quel à -15°C.

EXEMPLE 16

**Bis (phénylidèndioxy-2,2 acétyl)-2,3 éthylidène-4,6 β-D-glucose 2p.**
On reprend le mode opératoire de l'exemple 1, mais en utilisant du chlorure de phénylidèndioxy-2,2 acétyle, en lieu et place du chlorure de phénoxy-2 acétyle, et pendant un temps de réaction de 6h, pour obtenir l'intermédiaire 6p. Cet intermédiaire 6p a été directement hydrogénolysé à l'état brut pour donner le produit 2p sous forme d'une huile incolore, avec un rendement global de 15%. Le produit 2p a été conservé tel quel à -15°C.

EXEMPLE 17

**Bis (naphtylidèndioxy-2,2 acétyl)-2,3 éthylidène-4,6 β-D-glucose 2q.**
On reprend le mode opératoire de l'exemple 1, mais en utilisant du chlorure de naphtylidèndioxy-2,2 acétyle, en lieu et place du chlorure de phénoxy-2 acétyle, et pendant un temps de réaction de 9h, pour obtenir l'intermédiaire 6q. Cet intermédiaire 6q a été directement hydrogénolysé à l'état brut pour donner le produit 2q sous forme d'une huile incolore, avec un rendement global de 21%. Le produit 2q a été conservé tel quel à -15°C.

EXEMPLE 18

**Bis (benzylthio-2 acétyl)-2,3 éthylidène-4,6 β-D-glucose 2r.**
On reprend le mode opératoire de l'exemple 1, mais en utilisant du chlorure de benzylthio-2 acétyle, en lieu et place du chlorure de phénoxy-2 acétyle, et pendant un temps de réaction de 10h, pour obtenir l'intermédiaire **6r**. Cet intermédiaire **6r** a été directement hydrogénolysé à l'état brut pour donner le produit **2r** sous forme d'une résine jaune pâle, avec un rendement global de 34%. Le produit **2r** a été conservé tel quel à -15°C.

EXEMPLE 19

**Bis (phénylthio-2 acétyl)-2,3 éthylidène-4,6 β-D-glucose 2s.**
On reprend le mode opératoire de l'exemple 1, mais en utilisant du chlorure de phénylthio-2 acétyle, en lieu et place du chlorure de phénoxy-2 acétyle, et pendant un temps de réaction de 10h, pour obtenir l'intermédiaire **6s**. Cet intermédiaire 6s a été directement hydrogénolysé à l'état brut pour donner le produit **2s** sous forme d'une huile jaune, avec un rendement global de 37%. Le produit **2s** a été conservé tel quel à -15°C.

EXEMPLE 20

**Difuroyl-2,3 éthylidène-4,6 β-D-glucose 2t.**
On reprend le mode opératoire de l'exemple 1, mais en utilisant du chlorure de furoyle, en lieu et place du chlorure de phénoxy-2 acétyle, et pendant un temps de réaction de 4h, pour obtenir l'intermédiaire **6t**. Cet intermédiaire **6t** a été directement hydrogénolysé à l'état brut pour donner le produit **2t** sous forme d'une huile incolore, avec un rendement global de 41%. Le produit **2t** a été conservé tel quel à -15°C.

Les exemples 21 à 40 illustrent la préparation d'aglycones répondant à la formule 3.

EXEMPLE 21

**(Phénoxy-2 acétyl)4' déméthyl-4' épipodophyllotoxine 3a.**
A un mélange de dichlorométhane sec (17 l) et de pyridine anhydre (657 ml, soit 1,3 éq.), on ajoute de la déméthyl-4' épipodophyllotoxine (2,5 kg, soit 6,25 moles). Le milieu réactionnel a été refroidi à 0°C, puis on a additionné du chlorure de phénoxy-2 acétyle (1122 ml, soit 1,3 éq.) et la réaction s'est poursuivie à 20°C pendant 2h. Après contrôle par chromatographie en couche mince, le milieu a été lavé à la saumure. La phase organique a été séchée sur $MgSO_4$ puis concentrée sous pression réduite. L'huile obtenue a été cristallisée dans l'acétate d'éthyle. On a obtenu 3 kg d'un produit cristallin ( Rdt=90%). L'analyse structurale a donné les résultats suivants : PF=110-112°C; $[\alpha]_D^{22}$=-48° (c=1; $CHCl_3$); IR (Nujol) ( ν max): 3550 (OH), 1770 (C=O), 1603, 1383 et 1338; RMN[1]H 90MHz ( $CDCl_3$) (δ ppm): 6,95 à 7,55 (5H, 2m, H arom.); 6,89 ( 1H, s, H-5); 6,53 ( 1H, s, H-8); 6,39 (2H, s, H-2', 6'); 5,99 (2H, s, $O-CH_2-O$); 4,95 (2H, s, H benzylique); 4,82 ( 1H, d, $J_{4-3}$=4Hz, H-4); 4,64 ( 1H, d, $J_{1-2}$=5Hz, H-1); 4,33 (2H, m, H-11); 3,7 (6H, s, $OCH_3$); 3,29 ( 1H, dd, $J_{2-1}$)= 5 Hz, $J_{2-3}$=14,5 Hz, H-2) et 3,76 (2H, m, H-3, OH).

EXEMPLE 22

**(Naphtoxy-2 acétyl)4' déméthyl-4' épipodophyllotoxine 3b.**
En suivant le mode opératoire de l'exemple 21, mais en utilisant du chlorure de naphtoxy-2 acétyle en lieu et place du chlorure de phénoxy-2 acétyle, et un temps de réaction de 10h30, l'on a obtenu le produit **3b** sous forme d'une poudre jaune pâle amorphe ( Rdt=38%).

EXEMPLE 23

**[(Méthoxy-2 phénoxy)-2 acétyl]-4' diméthyl-4' épipodophyllotoxine 3c.**
En suivant le mode opératoire de l'exemple 21, mais en utilisant du chlorure de ( méthoxy-2 phénoxy)-2 acétyle en lieu et place du chlorure de phénoxy-2 acétyle, et un temps de réaction de 4h30, l'on a obtenu le produit **3c** sous forme d'un verre translucide (Rdt=80%).

EXEMPLE 24

**[(Méthoxy-4 phénoxy)-2 acétyl]-4' déméthyl-4' épipodophyllotoxine 3d.**

En suivant le mode opératoire de l'exemple 21, mais en utilisant du chlorure de ( méthoxy-4 phénoxy)-2 acétyle en lieu et place du chlorure de phénoxy-2 acétyle, et un temps de réaction de 5 h, l'on a obtenu le produit 3d sous forme d'une poudre blanche (Rdt=82%).

EXEMPLE 25

**[(Nitro-2 phénoxy)-2 acétyl]-4' déméthyl-4' épipodophyllotoxine 3e.**
En suivant le mode opératoire de l'exemple 21, mais en utilisant du chlorure de (nitro-2 phénoxy)-2 acétyle en lieu et place du chlorure de phénoxy-2 acétyle, et un temps de réaction de 2 h, l'on a obtenu le produit 3e sous forme d'une résine brun clair (Rdt=79%).

EXEMPLE 26

**[(Nitro-4 phénoxy)-2 acétyl]-4' déméthyl-4' épipodophyllotoxine 3f.**
En suivant le mode opératoire de l'exemple 21, mais en utilisant du chlorure de (nitro-4 phénoxy)-2 acétyle en lieu et place du chlorure de phénoxy-2 acétyle, et un temps de réaction de 2h 30, l'on a obtenu le produit 3f sous forme d'une poudre jaune foncée amorphe ( Rdt=73%).

EXEMPLE 27

[( Chloro-4 phénoxy)-2 acétyl]-4' déméthyl-4' épipodophyllotoxine 3g. En suivant le mode opératoire de l'exemple 21, mais en utilisant du chlorure de (chloro-4 phénoxy)-2 acétyle en lieu et place du chlorure de phénoxy-2 acétyle, et un temps de réaction de 3h 30, l'on a obtenu le produit 3g sous forme d'un verre jaune pâle (Rdt=77%).

EXEMPLE 28

**[( Dichloro-2,4 phénoxy)-2 acétyl]-4' déméthyl-4' épipodophyllotoxine 3h.**
En suivant le mode opératoire de l'exemple 21, mais en utilisant du chlorure de (dichloro-2,4 phénoxy)-2 acétyle en lieu et place du chlorure de phénoxy-2 acétyle, et un temps de réaction de 5 h, l'on a obtenu le produit 3h sous forme d'une poudre jaune ( Rdt=67%).

EXEMPLE 29

**[( Trichloro-2,4,5 phénoxy)-2 acétyl]-4' déméthyl-4' épipodophyllotoxine 3i.**
En suivant le mode opératoire de l'exemple 21, mais en utilisant du chlorure de ( trichloro-2,4,5 phénoxy)-2 acétyle en lieu et place du chlorure de phénoxy-2 acétyle, et un temps de réaction de 8 h, l'on a obtenu le produit 3i sous forme d'une poudre blanche ( Rdt=85%).

EXEMPLE 30

**[( Trichloro-2,4,6 phénoxy)-2 acétyl]-4' déméthyl-4' épipodophyllotoxine 3j.**
En suivant le mode opératoire de l'exemple 21, mais en utilisant du chlorure de ( trichloro-2,4,6 phénoxy)-2 acétyle en lieu et place du chlorure de phénoxy-2 acétyle, et un temps de réaction de 10 h, l'on a obtenu le produit 3j sous forme d'une poudre blanche amorphe (Rdt=81%).

EXEMPLE 31

**[(Fluoro-2 phénoxy)-2 acétyl]-4' déméthyl-4' épipodophyllotoxine 3k.**
En suivant le mode opératoire de l'exemple 21, mais en utilisant du chlorure de (fluoro-2 phénoxy)-2 acétyle en lieu et place du chlorure de phénoxy-2 acétyle, et un temps de réaction de 1h30, l'on a obtenu le produit 3k sous forme d'une poudre blanche amorphe (Rdt=63%).

EXEMPLE 32

**( Méthoxy-2 acétyl)-4' déméthyl-4' épipodophyllotoxine 3l.**
En suivant le mode opératoire de l'exemple 21, mais en utilisant du chlorure de méthoxy-2 acétyle en lieu et place du chlorure de phénoxy-2 acétyle, et un temps de réaction de 2 h, l'on a obtenu le produit 3l sous forme

d'un composé cristallisant spontanément à l'isolement (Rdt=84%); PF=200-203°C; $[\alpha]_D^{22}$=-59° ( c=1, $CHCl_3$);

ν

IR ( Nujol)( max): 3527 ( OH), 1769 (C=O), 1600, 1484 et 1132.

EXEMPLE 33

**(Ethoxy-2 acétyl)-4' déméthyl-4' épipodophyllotoxine 3m.**
En suivant le mode opératoire de l'exemple 21, mais en utilisant du chlorure d'éthoxy-2 acétyle en lieu et place du chlorure de phénoxy-2 acétyle, et un temps de réaction de 2 h, l'on a obtenu le produit **3m** sous forme d'une poudre blanche (Rdt=74%).

EXEMPLE 34

**(Benzoxy-2 acétyl)-4' déméthyl-4' épipodophyllotoxine 3n.**
En suivant le mode opératoire de l'exemple 21, mais en utilisant du chlorure de benzoxy-2 acétyle en lieu et place du chlorure de phénoxy-2 acétyle, et un temps de réaction de 4 h, l'on a obtenu le produit **3n** sous forme de microcristaux blancs, (Rdt=82%); PF=209-211°C; $[\alpha]_D^{22}$=-48° ( c=1, $CHCl_3$); IR ( Nujol)( ν max): 3550 ( OH), 1769 (C=O), 1600, 1484 et 1132.

EXEMPLE 35

**( Menthoxy-2 acétyl)-4' déméthyl-4' épipodophyllotoxine 3o.**
En suivant le mode opératoire de l'exemple 21, mais en utilisant du chlorure de menthoxy-2 acétyle en lieu et place du chlorure de phénoxy-2 acétyle, et un temps de réaction de 16 h, l'on a obtenu le produit **3o** sous forme d'une poudre blanche ( Rdt=33%).

EXEMPLE 36

**( Phénylidènedioxy-2,2 acétyl)-4' déméthyl-4' épipodophyllotoxine 3p.**
En suivant le mode opératoire de l'exemple 21, mais en utilisant du chlorure de phénylidènedioxy-2,2 acétyle en lieu et place du chlorure de phénoxy-2 acétyle, et un temps de réaction de 4 h, l'on a obtenu le produit **3o** sous forme d'une poudre jaune, amorphe ( Rdt=36%).

EXEMPLE 37

**( Naphtylidènedioxy-2,2 acétyl)-4' déméthyl-4' épipodophyllotoxine 3q.**
En suivant le mode opératoire de l'exemple 21, mais en utilisant du chlorure de naphtylidènedioxy-2,2 acétyle en lieu et place du chlorure de phénoxy-2 acétyle, et un temps de réaction de 4 h, l'on a obtenu le produit **3q** sous forme d'une poudre jaune, amorphe ( Rdt=30%).

EXEMPLE 38

**( Benzylthio-2 acétyl)-4' déméthyl-4' épipodophyllotoxine 3r.**
En suivant le mode opératoire de l'exemple 21, mais en utilisant du chlorure de benzylthio-2 acétyle en lieu et place du chlorure de phénoxy-2 acétyle, et un temps de réaction de 4 h, l'on a obtenu le produit **3r** sous forme d'une poudre blanche amorphe ( Rdt=69%).

EXEMPLE 39

**( Phénylthio-2 acétyl)-4' déméthyl-4' épipodophyllotoxine 3s.**
En suivant le mode opératoire de l'exemple 21, mais en utilisant du chlorure de phénylthio-2 acétyle en lieu et place du chlorure de phénoxy-2 acétyle, et un temps de réaction de 4h30, l'on a obtenu le produit **3s** sous forme d'une poudre blanche amorphe ( Rdt=77%).

EXEMPLE 40

**( Furoyl-4' déméthyl-4' épiponophyllotoxine 3t.**

En suivant le mode opératoire de l'exemple 21, mais en utilisant du chlorure de furoyle en lieu et place du chlorure de phénoxy-2 acétyle, et un temps de réaction de 2 h, l'on a obtenu le produit 3t sous forme d'une poudre blanche amorphe ( Rdt=83%).

Les exemples 41 à 60 illustrent la préparation de composés 1, par réaction d'un dérivé glycosylé 2 et d'une aglycone 3.

EXEMPLE 41

**Tris(phénoxy-2 acétyl)-2",3",4' éthylidène-4",6" β-D-glucopyranoside de déméthyl-4' épipodophyllotoxine 1a.**

On a introduit dans du dichlorométhane sec (18 l), la ( phénoxy-2 acétyl)-4' déméthyl-4' épipodophyllotoxine (3 kg, soit 5,62 moles) 3a issue de l'exemple 21 et le bis ( phénoxy-2 acétyl)-2,3 éthylidène-4,6 β-D-glucose 2a (3,2 kg, soit 6,74 moles ou 1,2 éq.), issu de l'exemple 1. Quand la température a été stabilisée à -18°C, on a additionné lentement du trifluorure de bore (1,73 l, soit 14,05 moles, soit 2,5 éq.). La réaction s'est poursuivie à -18°C pendant 2h, puis après contrôle par chromatographie en couche mince, on a ajouté de la pyridine (910 ml, soit 2 éq.). La solution a été lavée à l'eau, puis séchée sur $MgSO_4$ et concentrée sous pression réduite. Le produit brut a été filtré sur gel de silice [70-200 μ ; MeOH (2%)/$CH_2Cl_2$] et l'on a obtenu, après évaporation, 4,73 kg d'une poudre blanche, amorphe. Un échantillon a été recristallisé en vue d'une étude analytique. Les résultats sont les suivants :

Formule brute : $C_{53}H_{50}O_{19}$
Masse molaire : 990
Rendement : 85%
PF : 132-134°C
$[\alpha°]_D^{22}$ = -67 ( c=1; $CHCl_3$)

EXEMPLE 42

**Tris( naphtoxy-2 acétyl)-2",3",4' éthylinène-4",6" β-D-glucopyranosine de déméthyl-4' épiponophyllotoxine 1b.**

On a repris le mode opératoire de l'exemple 41, mais en utilisant du bis ( naphtoxy-2 acéty1)-2,3 éthylidène-4,6 β-D-glucose 2b et de la (naphtoxy-2 acétyl)-4' déméthyl-4' épipodophyllotoxine 3b, issus des exemples 2 et 22, respectivement, en lieu et place du ( phénoxy-2 acétyl)-2,3 éthylidène-4,6 β-D-glucose 2a, et de la ( phénoxy-2 acétyl)-4' déméthyl-4' épipodophyllotoxine 3a, pour obtenir le produit cherché.

Formule brute : $C_{65}H_{56}O_{19}$
Masse molaire : 1140
Rendement : 58%
PF : 136-139°C
$[\alpha°]_D^{22}$ = -60 ( c=1; $CHCl_3$)

EXEMPLE 43

**Tris[( méthoxy-2 phénoxy)-2 acétyl)]-2",3",4' éthylidène-4",6" β-D-glucopyranoside de déméthyl-4' épipodophyllotoxine 1c.**

On a repris le mode opératoire de l'exemple 41, mais en utilisant du bis[( méthoxy-2 phénoxy)-2 acétyl)]-2,3 éthylidène-4,6 β-D-glucose 2c et de la [( méthoxy-2 phénoxy)-2 acétyl)]-4' déméthyl-4' épipodophyllotoxine 3c, issus des exemples 3 et 23, respectivement, en lieu et place du ( phénoxy-2 acétyl)-2,3 éthylidène-4,6 β-D-glucose 2a, et de la ( phénoxy-2 acétyl)-4' déméthyl-4' épipodophyllotoxine 3a, pour obtenir le produit cherché.

Formule brute : $C_{56}H_{56}O_{22}$
Masse molaire : 1080
Rendement : 75%
PF : 113-115°C
$[\alpha°]_D^{22}$= -52 ( c=1; $CHCl_3$)

EXEMPLE 44

**Tris[(méthoxy-4 phénoxy)-2 acétyl)]-2", 3", 4' ethylidène-4", 6" β-D-glucopyranoside de déméthyl-4' épipodophyllotoxine 1d.**

On a repris le mode opératoire de l'exemple 41, mais en utilisant du bis [( méthoxy-4 phénoxy)-2 acétyl)]-2,3 éthylidène-4,6 β-D-glucose **2d** et de la [( méthoxy-4 phénoxy)-2 acétyl)]-4' déméthyl-4' épipodophyllotoxine **3d**, issus des exemples 4 et 24, respectivement, en lieu et place du ( phénoxy-2 acétyl)-2,3 éthylidène-4,6 β-D-glucose **2a**, et de la ( phénoxy-2 acétyl)-4' déméthyl-4' épipodophyllotoxine **3a**, pour obtenir le produit cherché.

Formule brute : $C_{56}H_{56}O_{22}$
Masse molaire : 1080
Rendement : 73%
PF : 107-109°C
$[\alpha°]_D^{22}$ = -61 ( c=1; $CHCl_3$)

EXEMPLE 45

**Tris[(nitro-2 phénoxy)-2 acétyl)]-2",3",4' éthylidène-4",6" β-D-glucopyranoside de déméthyl-4' épipodophyllotoxine 1e.**

On a repris le mode opératoire de l'exemple 41, mais en utilisant du bis[(nitro-2 phénoxy)-2 acétyl)]-2,3 éthylidène-4,6 β-D-glucose **2e** et de la [(nitro-2 phénoxy)-2 acétyl)]-4' déméthyl-4' épipodophyllotoxine **3e**, issus des exemples 5 et 25, respectivement, en lieu et place du ( phénoxy-2 acétyl)-2,3 éthylidène-4,6 β-D-glucose **2a**, et de la ( phénoxy-2 acétyl)-4' déméthyl-4' épipodophyllotoxine **3a**, pour obtenir le produit cherché.

Formule brute : $C_{53}H_{47}O_{25}N_3$
Masse molaire : 1125
Rendement : 79%
PF : 135-137°C
$[\alpha°]_D^{22}$ = -53 ( c=1; $CHCl_3$)

EXEMPLE 46

**Tris [(nitro-4 phénoxy)-2 acétyl)]-2",3",4' éthylidène-4",6" β-D-glucopyranoside de déméthyl-4' épipodophyllotoxine 1f.**

On a repris le mode opératoire de l'exemple 41, mais en utilisant du bis[(nitro-4 phénoxy)-2 acétyl)]-2,3 éthylidène-4,6 β-D-glucose **2f** et de la [(nitro-4 phénoxy)-2 acétyl)]-4' déméthyl-4' épipodophyllotoxine **3f**, issus des exemples 6 et 26, respectivement, en lieu et place du ( phénoxy-2 acétyl)-2,3 éthylidène-4,6 β-D-glucose **2a**, et de la ( phénoxy-2 acétyl)-4' déméthyl-4' épipodophyllotoxine **3a**, pour obtenir le produit cherché.

Formule brute : $C_{53}H_{47}O_{25}N_3$
Masse molaire : 1125
Rendement : 82%
PF : 143-145°C
$[\alpha°]_D^{22}$ = -72 ( c=1; $CHCl_3$)

EXEMPLE 47

**Tris[(chloro-4 phénoxy)-2 acétyl)]-2",3",4' éthylidène-4",6" β-D-glucopyranoside de déméthyl-4' épipodophyllotoxine 1q.**

On a repris le mode opératoire de l'exemple 41, mais en utilisant du bis[( chloro-4 phénoxy)-2 acétyl)]-2,3 éthylidène-4,6 β-D-glucose **2q** et de la [( chloro-4 phénoxy)-2 acétyl)]-4' déméthyl-4' épipodophyllotoxine **3q**, issus des exemples 7 et 27, respectivement, en lieu et place du ( phénoxy-2 acétyl)-2,3 éthylidène-4,6 β-D-glucose **2a**, et de la ( phénoxy-2 acétyl)-4' déméthyl-4' épipodophyllotoxine **3a**, pour obtenir le produit cherché.

Formule brute : $C_{53}H_{47}O_{19}Cl_3$
Masse molaire : 1093,5
Rendement : 79%
PF : 120-123°C
$[\alpha°]_D^{22}$ = -63 ( c=1: $CHCl_3$)

EXEMPLE 48

**Tris[( dichloro-2,4 phénoxy)-2 acétyl)]-2″,3″,4′ éthylidène-4″,6″ β-D-glucopyranosine de déméthyl-4′ épipodophyllotoxine 1h.**
On a repris le mode opératoire de l'exemple 41, mais en utilisant du bis[(dichloro-2,4 phénoxy)-2 acétyl)]-2,3 éthylidène-4,6 β-D-glucose 2h et de la [(dichloro-2,4 phénoxy)-2 acétyl)]-4′ déméthyl-4′ épipodophyllotoxine 3h, issus des exemples 8 et 28, respectivement, en lieu et place du ( phénoxy-2 acétyl)-2,3 éthylidène-4,6 β-D-glucose 2a, et de la (phénoxy-2 acétyl)-4′ déméthyl-4′ épipodophyllotoxine 3a, pour obtenir le produit cherché.

Formule brute : $C_{53}H_{44}O_{19}Cl_6$
Masse molaire : 1197
Rendement : 85%
PF : 115-117°C
$[\alpha°]_D^{22}$ = -51 ( c=1; $CHCl_3$)

EXEMPLE 49

**Tris[( trichloro-2,4,5 phénoxy)-2 acétyl)]-2″,3″,4′ éthylidène-4″,6″ β-D-glucopyranoside de déméthyl-4′ épipodophyllotoxine 1i.**
On a repris le mode opératoire de l'exemple 41, mais en utilisant du bis[( trichloro-2,4,5 phénoxy)-2 acétyl)]-2,3 éthylidène-4,6 β-D-glucose 2i et de la [( trichloro-2,4,5 phénoxy)-2 acétyl)]-4′ déméthyl-4′ épipodophyllotoxine 3i, issus des exemples 9 et 29, respectivement, en lieu et place du ( phénoxy-2 acétyl)-2,3 éthylidène-4,6 β-D-glucose 2a, et de la ( phénoxy-2 acétyl)-4′ déméthyl-4′ épipodophyllotoxine 3a, pour obtenir le produit cherché.

Formule brute : $C_{53}H_{41}O_{19}Cl_9$
Masse molaire : 1300,5
Rendement : 75%
PF : 126-128°C
$[\alpha°]_D^{22}$ = -43 ( c=1; $CHCl_3$)

EXEMPLE 50

**Tris[(trichloro-2,4,6 phénoxy)-2 acétyl)]-2″,3″,4′ éthylidène-4″,6″ β-D-glucopyranoside de déméthyl-4′ épipodophyllotoxine 1j.**
On a repris le mode opératoire de l'exemple 41, mais en utilisant du bis[(trichloro-2,4,6 phénoxy)-2 acétyl)]-2,3 éthylidène-4,6 β-D-glucose 2j et de la [(trichloro-2,4,6 phénoxy)-2 acétyl)]-4′ déméthyl-4′ épipodophyllotoxine 3j, issus des exemples 10 et 30, respectivement, en lieu et place du (phénoxy-2 acétyl)-2,3 éthylidène-4,6 β-D-glucose 2a, et de la (phénoxy-2 acétyl)-4′ déméthyl-4′ épipodophyllotoxine 3a, pour obtenir le produit cherché.

Formule brute : $C_{53}H_{41}O_{19}Cl_9$
Masse molaire : 1300,5
Rendement : 80%
PF : 120-122°C
$[\alpha°]_D^{22}$ = -10 (c=1; $CHCl_3$)

EXEMPLE 51

**Tris[(fluoro-2 phénoxy)-2 acétyl)]-2″, 3″, 4′ éthylidène-4″, 6″ β-D-glucopyranoside de déméthyl-4′ épipodophyllotoxine 1k.**
On a repris le mode opératoire de l'exemple 41, mais en utilisant du bis[(fluoro-2 phénoxy)-2 acétyl)]-2,3 éthylidène-4,6 β-D-glucose 2k et de la [(fluoro-2 phénoxy)-2 acétyl)]-4′ déméthyl-4′ épipodophyllotoxine 3k, issus des exemples 11 et 31, respectivement, en lieu et place du (phénoxy-2 acétyl)-2,3 éthylidène-4,6 β-D-glucose 2a, et de la (phénoxy-2 acétyl)-4′ déméthyl-4′ épipodophyllotoxine 3a, pour obtenir le produit cherché.

Formule brute : $C_{53}H_{47}O_{19}F_3$
Masse molaire : 1140
Rendement : 57%
PF : amorphe
$[\alpha°]_D^{22}$ = -58 (c=1; $CHCl_3$)

EXEMPLE 52

**Tris(méthoxy-2 acétyl)-2",3",4' éthylidène-4",6" β-D-glucopyranoside de déméthyl-4' épipodophyllotoxine 1l.**

On a repris le mode opératoire de l'exemple 41, mais en utilisant du bis(méthoxy-2 acétyl)-2,3 éthylidène-4,6 β-D-glucose **2l** et de la (méthoxy-2 acétyl)-4' déméthyl-4' épipodophyllotoxine **3l**, issus des exemples 12 et 32, respectivement, en lieu et place du (phénoxy-2 acétyl)-2,3 éthylidène-4,6 β-D-glucose **2a**, et de la (phénoxy-2 acétyl)-4' déméthyl-4' épipodophyllotoxine **3a**, pour obtenir le produit cherché.

Formule brute : $C_{38}H_{44}O_{19}$
Masse molaire : 804
Rendement : 82%
PF : 158-160
$[\alpha°]_D^{22}$ = -67 (c=1; $CHCl_3$)

EXEMPLE 53

**Tris(éthoxy-2 acétyl)-2", 3", 4' éthylidène-4", 6" β-D-glucopyranoside de déméthyl-4' épipodophyllotoxine 1m.**

On a repris le mode opératoire de l'exemple 41, mais en utilisant du bis(éthoxy-2 acétyl)-2,3 éthylidène-4,6 β-D-glucose **2m** et de l'(éthoxy-2 acétyl)-4' déméthyl-4' épipodophyllotoxine **3m**, issus des exemples 13 et 33, respectivement, en lieu et place du (phénoxy-2 acétyl)-2,3 éthylidène-4,6 β-D-glucose **2a**, et de la (phénoxy-2 acétyl)-4' déméthyl-4' épipodophyllotoxine **3a**, pour obtenir le produit cherché.

Formule brute : $C_{41}H_{50}O_{19}$
Masse molaire : 846
Rendement : 71%
PF : 195-198
$[\alpha°]_D^{22}$ = -64 (c=1; $CHCl_3$)

EXEMPLE 54

**Tris(benzoxy-2 acétyl)-2",3",4' éthylidène-4",6" β-D-glucopyranoside de déméthyl-4' épipodophyllotoxine 1n.**

On a repris le mode opératoire de l'exemple 41, mais en utilisant du bis(benzoxy-2 acétyl)-2,3 éthylidène-4,6 β-D-glucose **2n** et de la (benzoxy-2 acétyl)-4' déméthyl-4' épipodophyllotoxine **3n**, issus des exemples 14 et 34, respectivement, en lieu et place du (phénoxy-2 acétyl)-2,3 éthylidène-4,6 β-D-glucose **2a**, et de la (phénoxy-2 acétyl)-4' déméthyl-4' épipodophyllotoxine **3a**, pour obtenir le produit cherché.

Formule brute : $C_{56}H_{56}O_{19}$
Masse molaire : 1032
Rendement : 85%
PF : amorphe
$[\alpha°]_D^{22}$ = -46 (c=1; $CHCl_3$)

EXEMPLE 55

**Tris(menthoxy-2 acétyl)-2",3",4' éthylinène-4",6" β-D-glucopyranosine de déméthyl-4' épipodophyllotoxine 1o.**

On a repris le mode opératoire de l'exemple 41, mais en utilisant du bis(menthoxy-2 acétyl)-2,3 éthylidène-4,6 β-D-glucose **2o** et de la (menthoxy-2 acétyl)-4' déméthyl-4' épipodophyllotoxine **3o**, issus des exemples 15 et 35, respectivement, en lieu et place du (phénoxy-2 acétyl)-2,3 éthylidène-4,6 β-D-glucose **2a**, et de la (phénoxy-2 acétyl)-4' déméthyl-4' épipodophyllotoxine **3a**, pour obtenir le produit cherché.

Formule brute : $C_{65}H_{92}O_{19}$
Masse molaire : 1300,5
Rendement :68%
PF : amorphe
$[\alpha°]_D^{22}$ = -97 (c=1; $CHCl_3$)

EXEMPLE 56

**Tris(phénylidendioxy-2,2 acétyl)-2",3",4' éthylidène-4",6" β-D-glucopyranoside de déméthyl-4' épipodophyllotoxine 1p.**

On a repris le mode opératoire de l'exemple 41, mais en utilisant du bis(phénylidendioxy-2 acétyl)-2,3 éthylidène-4,6 β-D-glucose 2p et de la (phénylidendioxy-2 acétyl)-4' déméthyl-4' épipodophyllotoxine 3p, issus des exemples 16 et 36, respectivement, en lieu et place du (phénoxy-2 acétyl)-2,3 éthylidène-4,6 β-D-glucose 2a, et de la (phénoxy-2 acétyl)-4' déméthyl-4' épipodophyllotoxine 3a, pour obtenir le produit cherché.

Formule brute : $C_{53}H_{44}O_{22}$
Masse molaire : 1032
Rendement :58%
PF : amorphe
$[\alpha°]_D^{22}$ = -35 (c=1; $CHCl_3$)

EXEMPLE 57

**Tris(naphtylidendioxy-2,2 acétyl)-2",3",4' éthylidène-4",6" β-D-glucopyranoside de déméthyl-4' épipodophyllotoxine 1q.**

On a repris le mode opératoire de l'exemple 41, mais en utilisant du bis(naphtylidendioxy-2,2 acétyl)-2,3 éthylidène-4,6 β-D-glucose 2q et de la (naphtylidendioxy-2,2 acétyl)-4' déméthyl-4' épipodophyllotoxine 3q, issus des exemples 17 et 37, respectivement, en lieu et place du (phénoxy-2 acétyl)-2,3 éthylidène-4,6 β-D-glucose 2a, et de la (phénoxy-2 acétyl)-4' déméthyl-4' épipodophyllotoxine 3a, pour obtenir le produit cherché.

Formule brute : $C_{65}H_{50}O_{22}$
Masse molaire : 1182
Rendement :64%
PF : 157-159
$[\alpha°]_D^{22}$ = -40 (c=1; $CHCl_3$)

EXEMPLE 58

**Tris(benzylthio-2 acétyl)-2",3",4' éthylidène-4",6" β-D-glucopyranoside de déméthyl-4' épipodophyllotoxine 1r.**

On a repris le mode opératoire de l'exemple 41, mais en utilisant du bis(benzylthio-2 acétyl)-2,3 éthylidène-4,6 β-D-glucose 2r et de la (benzylthio-2 acétyl)-4' déméthyl-4' épipodophyllotoxine 3r, issus des exemples 18 et 38, respectivement, en lieu et place du (phénoxy-2 acétyl)-2,3 éthylidène-4,6 β-D-glucose 2a, et de la (phénoxy-2 acétyl)-4' déméthyl-4' épipodophyllotoxine 3a, pour obtenir le produit cherché.

Formule brute : $C_{56}H_{56}O_{16}S_3$
Masse molaire : 1080
Rendement :49%
PF : amorphe
$[\alpha°]_D^{22}$ = -48 (c=1; $CHCl_3$)

EXEMPLE 59

**Tris(phénylthio-2 acétyl)-2",3",4' éthylidène-4",6" β-D-glucopyranoside de déméthyl-4' épipodophyllotoxine 1s.**

On a repris le mode opératoire de l'exemple 41, mais en utilisant du bis(phénylthio-2 acétyl)-2,3 éthylidène-4,6 β-D-glucose 2s et de la (phénylthio-2 acétyl)-4' déméthyl-4' épipodophyllotoxine 3s, issus des exemples 19 et 39, respectivement, en lieu et place du (phénoxy-2 acétyl)-2,3 éthylidène-4,6 β-D-glucose 2a, et de la (phénoxy-2 acétyl)-4' déméthyl-4' épipodophyllotoxine 3a, pour obtenir le produit cherché.

Formule brute : $C_{53}H_{50}O_{16}S_3$
Masse molaire : 1038
Rendement :82%
PF : amorphe
$[\alpha°]_D^{22}$ = -60 (c=1; $CHCl_3$)

EXEMPLE 60

**Trifluroyl-2",3",4' éthylidène-4",6" β-D-glucopyranoside de déméthyl-4' épipodophyllotoxine 1t.**

On a repris le mode opératoire de l'exemple 41, mais en utilisant du difuroyl-2,3 éthylidène-4,6 β-D-glucose 2t et de la furoyl-4' déméthyl-4' épipodophyllotoxine 3t, issus des exemples 20 et 30, respectivement, en lieu et place du (phénoxy-2 acétyl)-2,3 éthylidène-4,6 β-D-glucose 2a, et de la (phénoxy-2 acétyl)-4' déméthyl-4' épipodophyllotoxine 3a, pour obtenir le produit cherché.

Formule brute : $C_{44}H_{38}O_{19}$
Masse molaire : 870
Rendement :89%
PF : 278-280
$[\alpha°]_D^{22}$ = -15 (c=1; $CHCl_3$)

Les exemples suivants illustrent l'utilisation des composés 1 pour préparer l'éthylidène-4,6 β-D-glucopyranoside de déméthyl-4' épipodophyllotoxine 1'.

EXEMPLE 61

**Préparation de 1' à partir du tris(phénoxy-2 acétyl)-2",3",4' éthylidène-4,6 β-D-glucopyranoside de déméthyl-4' épipodophyllotoxine 1a.**

On a mis en suspension du tris(phénoxy-2 acétyl)-2"-3"-4' éthylidène-4",6" β-D-glucopyranoside de déméthyl-4' épipodophyllotoxine **1a** issu de l'exemple 41 (2,2kg, soit 2,22 moles) dans du méthanol (25 l), additionné de tétrahydrofuranne (2,5 l). On a ajouté de l'acétate de zinc (1,1 kg), puis le milieu réactionnel a été porté au reflux pendant 5h. Après contrôle par chromatographie en couche mince, la solution a été concentrée et le concentrat introduit dans du dichlorométhane (25 l) additionné d'un mélange à 6% d'acide acétique dans le méthanol (3,5 l). La solution a été lavée à l'eau, séchée sur $MgSO_4$, puis évaporée sous pression réduite, jusqu'à l'obtention d'une huile épaisse qui a été soumise telle quelle à la chromatographie sur gel de silice [20-45 µm; MEOH (2 à 5%)/$CH_2Cl_2$]. M=979 g; Rdt=75%; PF=257-266°C ($CH_2Cl_2$/$Et_2O$; prismes maclés); $[\alpha]_D^{22}$=-103°(c=0,6; $CHCl_3$).

EXEMPLE 62

**Préparation de 1' à partir du tris(naphtoxy-2 acétyl)-2",3",4' éthylidène-4,6 β-D-glucopyranoside de déméthyl-4' épipodophyllotoxine 1b.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 6 h. Le composé obtenu est en tout point identique, d'une part, à celui obtenu dans l'exemple 61, et d'autre part à un échantillon de référence, avec un rendement de 70% en produit cristallisé.

EXEMPLE 63

**Préparation de 1' à partir du tris[(méthoxy-2 phénoxy)-2 acétyl)]-2",3",4' éthylidène-4,6 β-D-glucopyranoside de déméthyl-4' épipodophyllotoxine 1c.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 5 h. Le composé obtenu est en tout point identique, d'une part, à celui obtenu dans l'exemple 61, et d'autre part à un échantillon de référence, avec un rendement de 78% en produit cristallisé.

EXEMPLE 64

**Préparation de 1' à partir du tris[(méthoxy-4 phénoxy)-2 acétyl)]-2",3",4' éthylidène-4,6 β-D-glucopyranoside de déméthyl-4' épipodophyllotoxine 1d.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 5 h. Le composé obtenu est en tout point identique, d'une part, à celui obtenu dans l'exemple 61, et d'autre part à un échantillon de référence, avec un rendement de 85% en produit cristallisé.

EXEMPLE 65

**Préparation de 1' à partir du tris[(nitro-2 phénoxy)-2 acétyl)]-2",3",4' éthylidène-4,6 β-D-glucopyranoside de déméthyl-4' épipodophyllotoxine 1e.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 80% en produit cristallisé.

EXEMPLE 66

**Préparation de 1' à partir du tris[(nitro-4 phénoxy)-2 acétyl)]-2",3",4' éthylidène-4,6 β-D-glucopyranoside de déméthyl-4 épipodophyllotoxine 1f.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 87% en produit cristallisé.

EXEMPLE 67

**Préparation de 1' à partir du tris[(chloro-4 phénoxy)-2 acétyl)]-2",3",4' éthylidène-4,6 β-D-glucopyranoside de déméthyl-4' épipodophyllotoxine 1g.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 82% en produit cristallisé.

EXEMPLE 68

**Préparation de 1' à partir du tris[(dichloro-2,4 phénoxy)-2 acétyl)]-2",3",4' éthylidène-4,6 β-D-glucopyranoside de déméthyl-4' épipodophyllotoxine 1h.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 82% en produit cristallisé.

EXEMPLE 69

**Préparation de 1' à partir du tris[(trichloro-2,4,5 phénoxy)-2 acétyl)]-2",3",4' éthylidène-4,6 β-D-glucopyranoside de déméthyl-4' épipodophyllotoxine 1i.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 85% en produit cristallisé.

EXEMPLE 70

**Préparation de 1' à partir du tris[(trichloro-2,4,6 phénoxy)-2 acétyl)]-2",3",4" éthylidène-4,6 β-D-glucopyranoside de déméthyl-4' épipodophyllotoxine 1j.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 82% en produit cristallisé.

EXEMPLE 71

**Préparation de 1' partir du tris[(fluoro-2-phénoxy)-2 acétyl)]-2",3",4' éthylidène-4,6 β-D-glucopyranoside de déméthyl-4' épipodophyllotoxine 1k.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 91% en produit cristallisé.

EXEMPLE 72

**Préparation de 1' à partir du tris (méthoxy-2 acétyl)-2",3",1' éthylidène-4,6 β-D-glucopyranoside de déméthyl-4' épipodophyllotoxine 1l.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 70% en produit cristallisé.

EXEMPLE 73

**Préparation de 1' à partir du tris(éthoxy-2 acétyl)-2",3",4' éthylidène-4,6 β-D-glucopyranoside de déméthyl-4' épipodophyllotoxine 1m.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 61% en produit cristallisé.

EXEMPLE 74

**Préparation de 1' à partir du tris(benzoxy-2 acétyl)-2",3",4' éthylidène-4,6 β-D-glucopyranoside de déméthyl-4' épipodophyllotoxine 1n.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 86% en produit cristallisé.

EXEMPLE 75

**Préparation de 1' à partir du tris( menthoxy-2 acétyl)-2",3",4' éthylidène-4,6 β-D-glucopyranoside de déméthyl-4' épipodophyllotoxine 1o.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La réaction a été jugée complète par chromatographie en couche mince au bout de 4 h. Le composé obtenu est en tout point identique, d'une part, à celui obtenu dans l'exemple 61, et d'autre part à un échantillon de référence, avec un rendement de 15% en produit cristallisé.

EXEMPLE 76

**Préparation de 1' à partir du tris(phénylidendioxy-2,2 acétyl)2",3",4' éthylidène-4,6 β-D-glucopyranoside de déméthyl-4' épipodophyllotoxine 1p.**

On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine **1a**. La

réaction a été jugée complète par chromatographie en couche mince au bout de 1 h. Le composé obtenu est en tout point identique, d'une part, à celui obtenu dans l'exemple 61, et d'autre part à un échantillon de référence, avec un rendement de 79% en produit cristallisé.

EXEMPLE 77

**Préparation de 1' à partir du tris(naphtylidendioxy-2,2 acétyl)-2",3",4' éthylidène-4,6 β-D-glucopyranoside de déméthyl-4' épipodophyllotoxine 1q.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 1 h. Le composé obtenu est en tout point identique, d'une part, à celui obtenu dans l'exemple 61, et d'autre part à un échantillon de référence, avec un rendement de 90% en produit cristallisé.

EXEMPLE 78

**Préparation de 1' à partir du tris( benzylthio-2 acétyl)2",3",4' éthylidène-4,6 β-D-glucopyranoside de déméthyl-4' épipodophyllotoxine 1r.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a . La réaction a été jugée complète par chromatographie en couche mince au bout de 6 h. Le composé obtenu est en tout point identique, d'une part, à celui obtenu dans l'exemple 61, et d'autre part à un échantillon de référence, avec un rendement de 64% en produit cristallisé.

EXAMPLE 79

**Préparation de 1' à partir du tris(phénylthio-2 acétyl)2",3",4' éthylidène-4,6 β-D-glucopyranoside de déméthyl-4' épipodophyllotoxine 1s.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 7 h. Le composé obtenu est en tout point identique, d'une part, à celui obtenu dans l'exemple 61, et d'autre part à un échantillon de référence, avec un rendement de 55% en produit cristallisé.

EXEMPLE 80

**Préparation de 1' à partir du trifuroyl-2",3",4" éthylidène-4,6 β-D-glucopyranoside de déméthyl-4' épipodophyllotoxine 1t.**
On a repris le mode opératoire de l'exemple 61, mais en utilisant le triester cité en titre en lieu et place du tris(phénoxy-2 acétyl)-2",3",4' éthylidène-4,6 β-D-glucopyranoside de déméthyl-4' épipodophyllotoxine 1a. La réaction a été jugée complète par chromatographie en couche mince au bout de 24 h. Le composé obtenu est en tout point identique, d'une part, à celui obtenu dans l'exemple 61, et d'autre part à un échantillon de référence, avec un rendement de 22% en produit cristallisé.

## Revendications

1. Procédé de préparation de composés répondant à la formule 1

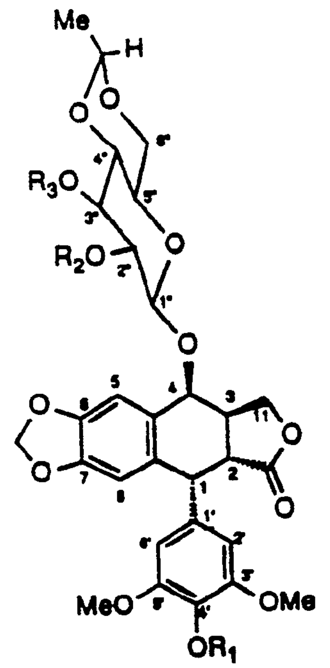

1

dans laquelle $R_1$, $R_2$ et $R_3$, identiques ou différents, représentent des radicaux acyles dans lesquels le carbone en $\alpha$ de la fonction carbonyle porte au coins un hétéroatome choisi parmi O et S, caractérisé en ce qu' il comporte les étapes suivantes :

1) préparation d'un dérivé glycosylé répondant à la formule 2,

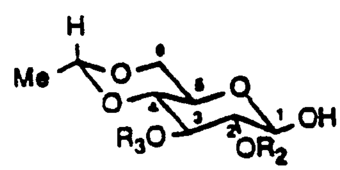

2

dans laquelle $R_2$ et $R_3$ ont la signification donnée ci-dessus, par réaction d'au moins un chlorure d'acide $R_2Cl$ et/ou $R_3Cl$ dans lequel le carbone en $\alpha$ de la fonction carbonyle porte au moins un hétéroatome choisi parmi O et S avec un dérivé glycosylé répondant à la formule 2'

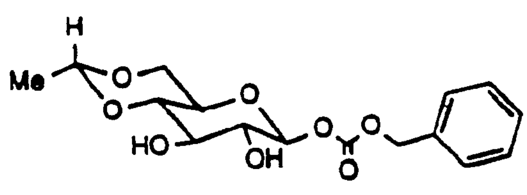

2'

suivie d'une hydrogénolyse du composé intermédiaire 6 obtenu, catalysée par du charbon palladié.

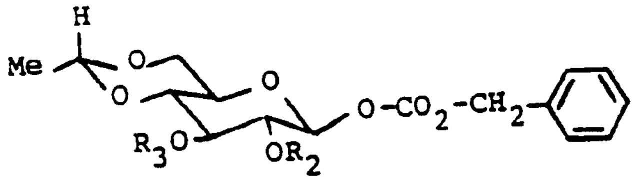

6

2) préparation d'une aglycone répondant à la formule 3,

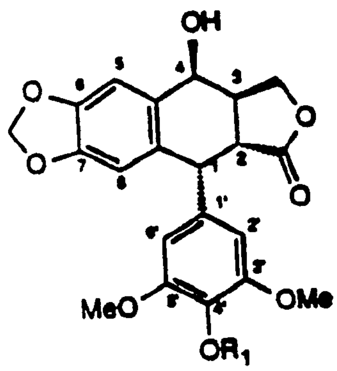

3

dans laquelle $R_1$ a la signification donnée ci-dessus, par réaction de déméthyl-4′ épipodophyllotoxine avec un chlorure d'acide $R_1Cl$ dans lequel le carbone en α de la fonction carbonyle porte au moins un hétéroatome choisi parmi O et S, la réaction étant effectuée en milieu neutre; l'ordre dans lequel les étapes 1) et 2) sont effectuées étant indifférent;

3) couplage d'un dérivé glycosylé 2 avec une aglycone 3 en présence d'un acide de Lewis en solution dans un hydrocarbure chloré.

2. Procédé selon la revendication 1, caractérisé en ce que les chlorures d'acide utilisés, identiques ou différents, sont choisis parmi les chlorures d'acides dont le radical acyle $R_1$, $R_2$ ou $R_3$ répond aux formules **4a** à **4h** suivantes :

(4a)

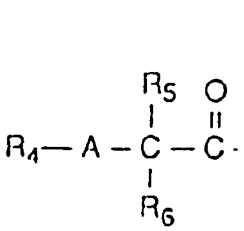

(4b)

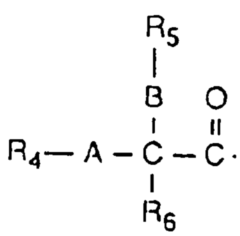

(4c)

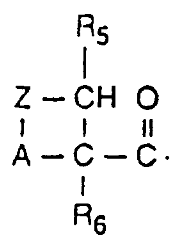

(4d)

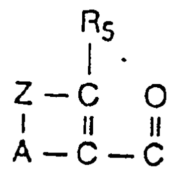

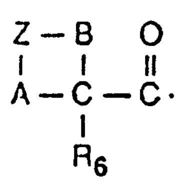

(4e)

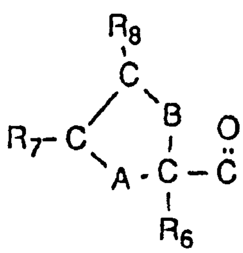

(4f)

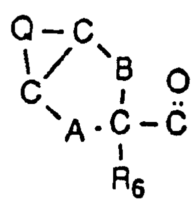

(4g)

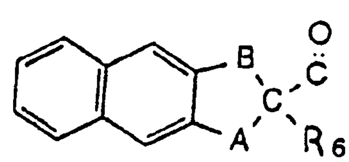

(4h)

dans lesquelles

– A et B, identiques ou différents, représentent chacun un hétéroatome divalent d'oxygène et/ ou de soufre,

– $R_4$, $R_5$, $R_6$, $R_7$, $R_8$, identiques ou différents, représentent chacun, soit un atome d'hydrogène, soit un radical alkyle ayant de 1 à 8 atomes de carbone, saturé, monoinsaturé ou polyinsaturé, linéaire ou ramifié, soit un radical alkyle choisi parmi les motifs suivants : Ar-$(CH_2)_n$- ( où n est un nombre entier égal à 1 ou 2, et Ar est un benzène, un naphtalène ou un anthracène), $Ar_2CH$- ( où Ar est un benzène ou un naphtalène), soit un noyau aromatique choisi parmi les motifs répondant aux formules **5a** et **5c** suivantes

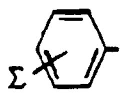
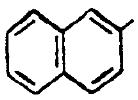
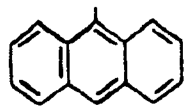

(5a) (5b) (5c)

où Σ représente un ensemble de 1 à 4 substituants identiques ou différents choisis indépendamment parmi les motifs ou atomes suivants : OMe, OEt, F, Cl, Br, I, $NO_2$.

– Z et Q identiques ou différents, représentent chacun un motif divalent choisi parmi -$(CH_2)_n$-, -$CH(CH_3)$-$CH_2$-, -$CH_2$-$CH(CH_3)$-, -$CH(CH_3)$-$CH(CH_3)$-, -$CH(C_2H_5)$-$CH_2$-, -$CH_2$-$CH(C_2H_5)$-, CH=$C(CH_3)$-, -$C(CH_3)$=CH-, -CH=CH-$(CH_2)_m$, -CH=CH-CH=CH-, n étant un nombre entier allant de 1 ) 4 inclus et m un nombre entier égal à 1 ou 2, un radical 1,2-phénylène, 2,3-naphtylène, 2,3-anthrylène, un motif tétravalent répondant à la formule suivante:

=$C(R_9)$-$CR_{10}$=$CR_{11}$-$CR_{12}$=, où $R_9$, $R_{10}$, $R_{11}$ et $R_{12}$ sont des radicaux alkyles ayant de 1 à 6 atomes de carbone ou des hétéroatomes d'oxygène, de soufre, d'halogènes ( F, Cl, Br, I), ou d'azote.

**3.** Procédé selon l'une des revendications 1 ou 2, caractérisé en ce que l'un au moins des radicaux acyles est un radical aryloxyacétyle $R_4$-O-$CH_2$-C=0, $R_4$ étant choisi parmi les noyaux phénoxy, naphtoxy, anthroxy et phénanthroxy, portant éventuellement de 1 à 4 substituants choisis parmi OMe, OEt, F, Cl, Br, I, $NO_2$.

**4.** Procédé selon l'une des revendications 1 à 3, caractérisé en ce que l'un au moins des radicaux acyles est choisi parmi les radicaux alcoxyacétyles $R_4$-$OCH_2$-C=0, $R_4$ étant choisi parmi les radicaux méthyle, éthyle, benzyle ou menthyle.

**5.** Procédé selon l'une quelconque des revendications 1 à 4, caractérisé en ce que l'un au moins des radicaux acyles est

choisi parmi les radicaux arylidènedioxyantyle 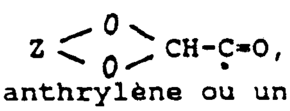 , Z représentant un phénylène, un naphtylène, un anthrylène ou un phénanthrylène.

**6.** Procédé selon l'une quelconque des revendications 1 à 5, caractérisé en ce que l'un au moins des radicaux acyles est choisi parmi les radicaux alcoylthioacétyles $R_4$-S-$CH_2$-C=0, $R_4$ étant choisi parmi les radicaux méthyle, éthyle, benzyle.

**7.** Procédé selon l'une quelconque des revendications 1 à 6, caractérisé en ce que l'un au moins des radicaux acyles est choisi parmi les radicaux arylthioacétyle $R_4$-S-$CH_2$-C=0, $R_4$ étant choisi parmi les radicaux phényle, naphtyle, anthracényle.

**8.** Procédé selon l'une quelconque des revendications 1 à 7, caractérisé en ce que l'un au moins des radicaux acyles est choisi parmi les radicaux 2-furoyle ou 2-thénoyle.

**9.** Tris acétyl-2",3",4'-β-D-glucopyranoside de déméthyl-4' épipodophyllotoxine obtenue par un procédé selon l'une quelconque des revendications 1 à 8.

10. Tris acétyl-2",3",4'-β-D-glucopyranoside de déméthyl-4' épipodophyllotoxine répondant à la formule 1, dans laquelle $R_1$, $R_2$ et $R_3$ identiques ou différents représentent un radical acyle ayant au moins un hétéroatome choisi parmi D et S sur le carbone en de la fonction carbonyle.

11. Tris acétyl-2",3",4'-β-D-glucopyranoside de déméthyl-4' épipodophyllotoxine répondant à la formule 1, dans laquelle $R_1$, $R_2$ et $R_3$ ont la signification donnée dans les revendications 2 à 9.

12. Utilisation d'un composé 1 selon l'une des revendications 9, 10 ou 11, pour la préparation de β-D-glucopyranoside de déméthyl-4' épipodophyllotoxine répondant à la formule 1'.

13. Dérivé glycosylé répondant à la formule 2, caractérisé en ce que $R_2$ et $R_3$, identiques ou différents, sont des radicaux acyles ayant la signification donnée dans les revendications 1 à 9.

14. Composé répondant à la formule 3, caractérisé en ce que le radical $R_1$ est un radical acyle ayant la signification donnée dans les revendications 1 à 9.